# EUROPEAN PATENT SPECIFICATION

(11) **EP 1 776 935 B1**
(45) Date of publication and mention of the grant of the patent: **12.08.2009**
(21) Application number: 06255458.9
(22) Date of filing: 24.10.2006
(51) Int. Cl.: A61F 2/40

(54) **Prosthetic glenoid component**
Glenoidale prosthetische Komponente
Composant de prosthese glenoide

(30) Priority: 24.10.2005 GB 0521634
(43) Date of publication of application: 25.04.2007
(73) Proprietor: BENOIST GIRARD SAS, 14201 Hérouville-Saint-Clair Cédex (FR)
(72) Inventor: Geary, Cathal, Tralee County Kerry (IE); Birkinshaw, Colin, County Limerick (IE); Jones, Eric, Country Limerick (IE)
(74) Representative: Bridge-Butler, Alan James

(56) References cited:
- EP-A- 1 520 561
- DE-A1- 2 041 929
- FR-A- 2 825 263
- FR-A1- 2 683 142
- US-A- 5 593 448
- US-A1- 2004 220 673
- US-B1- 6 364 910

## Description

This invention relates to a prosthetic glenoid component and to a method of attaching it in place.

There are various aspects to the invention, one aspect relating to a glenoid component having a compliant bearing surface for articulating with a prosthetic humeral head and a method of fixing such a glenoid component to a scapula in an implant procedure.

Traditional unconstrained total shoulder arthroplasty, based on the Neer Mark II system dating back to 1974, has proven to be a highly successful procedure with good results in more than 85% of shoulders evaluated at early and mid-term follow up. Despite this success, glenoid wear and loosening and glenohumeral instability still threaten long-term component survival and represent the primary sources of complications.

Observations on retrieved glenoid components clearly indicate polyethylene wear and damage is a key factor in determining the long-term survivorship of total shoulder arthroplasty. UHMWPE relies on the inherent low frictional and wear properties of the material rather than the mode of lubrication. In contrast the soft articular cartilage of the natural joint promotes fluid film lubrication and almost zero wear throughout most of its operation. A low modulus polyurethane glenoid bearing surface fulfils a similar function to cartilage, achieving continuous fluid film lubrication between the articulating surfaces and reducing both friction and ear. [1. Unsworth, A., Roberts, B., and Thompson, J.C. The application of soft layer lubrication to hip prosthesis. J Bone Jt Surg., 1981, 63-B, 297. 2. Unsworth, A., Pearcy, M.J., White, E.F.T., and White, G. Soft layer lubrication of artificial hip with reduced wear rates, joints. Proceedings of the International Conference on tribology, friction, lubrication, and wear, 50 years on, 1987, Mechanical Engineering Publications Ltd., London, 715-724. 3. Unsworth, A., Pearcy, M.J., White, E.F.T., and White, G. Frictional properties of artificial hip joints. J. Engng in Medicine, 1988, 17(3), 101-104.] The promotion of a continuous film of lubricant may be attributed to conventional elastohydrodynamic lubrication and squeeze film effects, micro-elastohydrodynamic lubrication and local deformation of the low elastic modulus layer. Contact width is the predominant factor controlling the thickness of the fluid film generated and can be optimised by appropriate selection of layer thickness and radial mismatch. Renewed confidence in glenoid components through lower wear rates and longer survivability will allow earlier surgical intervention before glenoid bone stock is compromised and adversely affects long-term survival. The preoperative state of unsuccessful total shoulder arthroplasty is often gross destruction of the glenohumeral joint. Considerable variability in the rate of disease progression and in the morphology of glenoid bone as a result of gross destruction has also been reported. Minimising complications associated with such variability through early intervention can improve the outcome of total shoulder arthroplasty. Timing of the operating procedure is therefore critical.

One of the main considerations in glenoid prosthesis design is radial mismatch with the humeral head. The requirements for radial mismatch are contradictory however. In the natural glenoid, the deformable nature or compliance of the articular cartilage and glenoid labrum allow translations and provide shock absorption for eccentric loads. There is sufficient evidence that the radial mismatch between the bony articulating surfaces is compensated for by varied cartilage thickness effectively making the glenohumeral joint bearing surfaces closely congruent. Flatlow et al. found a radial mismatch of less than 0.1mm when they examined the cartilage articular surfaces.

Standard polyethylene prosthetic surfaces are much stiffer however and similar shock absorption must be provided through radial mismatch between the humeral and glenoid components. This allows some translation before the humeral head causes rim loading which introduces large rocking moments and subsequent loosening. However, increasing radial mismatch has the adverse effect of decreasing contact area and increasing contact pressures and wear rate.

Glenoid designs using harder materials like UHMWPE are forced into a compromise in the consideration of radial mismatch. Only alternative low modulus bearing materials that simulate cartilage more closely can resolve these contradictory requirements.

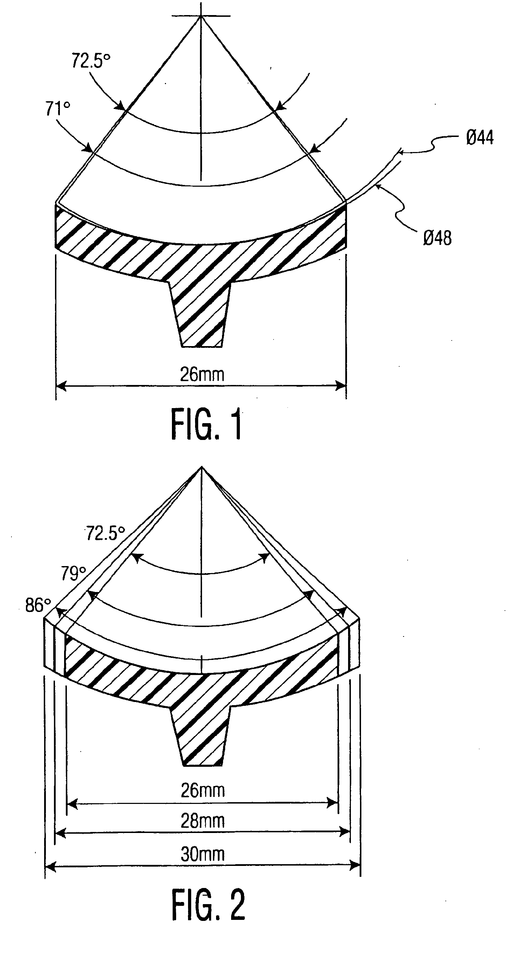

Glenohumeral instability is one of the most common complications following total shoulder arthroplasty. Often attributable to abnormal capsular tension, rotator cuff dysfunction, decreased proprioceptive feedback and joint stuffing due to glenoid component thickness, instability is an unavoidable complication which experiences very modest success rates from surgical treatment. This inevitability of compromised joint stability makes restoring the effective glenoid arc or balance stability angle (BSA) of the glenoid even more important. The balance stability angle is the maximal angle that the net humeral joint reaction force can make before dislocation occurs, as shown in Figure 1.

In the anatomical glenoid the bone, cartilage and labrum all contribute to the BSA. Recent studies of retrieved glenoid components [4.Weldon, E.J., Scarlat, M.M., Lee, S.B., and Matsen, F.A. Intrinsic stability of unused and retrieved polyethylene glenoid components. J Shoulder Elbow Surg. 2001, 10(5), 474-481. Scarlat, M.M., Matsen, F.A. 2001. Observations on retrieved polyethylene glenoid components. J. Arthroplasty 2001, Vol. 16 No.6, pp. 795-801.] have concluded that in vivo damage to the surface geometry of polyethylene glenoid components compromises their contribution to glenohumeral stability. In particular, rim erosion of the glenoid component tends to flatten the glenoid contour effectively diminishing the balance stability angle. In an effort to explain anterior and posterior rim deformation of retrieved glenoid components [Friedman, R.J. 1992. Glenohumeral translation after total should arthroplasty. J Shoulder Elbow Surg, 1992, Vol.1, No.6, pp.312-316.] attempted to define the amount of antero-posterior translation after total shoulder arthroplasty with matching radii of curvature components. The average total translation was 4mm mainly in a posterior direction. It was recently confirmed [Hertel, R., Ballmer, F.T. 2003. Observations on retrieved glenoid components. J Arthroplasty. Vol.18, No.3, 2003, pp.361-366.] intraoperatively that rim deformation was caused by direct contact with the humeral metaphysis (neck region and tuberosities). Most likely a combination of the two modes causes rim deformation and subsequent reduction in balance stability angles.

The instability problem must be addressed to improve long-term results and ensure that glenoid components that have achieved good fixation do not require revision. Soft tissue balancing intraoperatively is outside the scope of glenoid design so the intrinsic stability resulting from component geometry needs to be addressed. As illustrated in Figure 1 there are two ways in which the BSA can be increased. The first is by reducing the glenoid diameter to match the humeral head e.g. 44mm and having no radial clearance with an increase in BSA OF 1.5°. This also increases the risk of cavitation. The second and by far the most effective method is to increase the glenoid width and improve stability by up to 20%.

While improving the balance stability angle will prevent easy dislocation the force that resists subluxation is located away from the component midlines transmitting large bending moments to component fixation. Clearly an improvement in component fixation is required in parallel with improvement in component stability.

It is a further objective of the invention to provide a new method of attaching an all polymer glenoid prosthesis that is bio-mechanically compatible and results in improved fixation. Bone is constantly remodelling itself through a delicate balance between an osteogenic (bone forming) and osteoclastic (bone removing) process. These processes will respond to changes in the static and dynamic stress applied to bone. Overloading the implant-bone interface or shielding it from load transfer may result in bone resorption and subsequent loosening. Predicting the thresholds of such activity is impossible therefore focussing stress transfer through component design runs a very high risk of inducing bone resorption leading to component loosening and subsequent failure. Differences in stress transfer resulting from implantation are hypothesised to be the driving force behind adaptive changes in the bone leading to component loosening. It is therefore one aim of this invention to incur minimal bone resection allowing near normal stress transfer to the glenoid vault to take place.

The high incidence of radiolucencies in current glenoid fixation methods demonstrates the need for alternative fixation designs based on the kinematics and dynamic load transfer characteristics of the joint. It is particularly important to remember that total shoulder arthroplasty is not performed on healthy joints and disease related morphological changes to glenoid bone must be taken into consideration.

While a complete understanding of the bone remodelling system is lacking the close relationship between bone mineralisation density and stress transfer patterns has been well documented. The two most common indications for total shoulder arthroplasty are primary glenohumeral osteoarthritis (GHOA) and rheumatoid arthritis (RA). In GHOA the humeral head tends to translate posteriorly resulting in excessive load transfer to the posterior glenoid thereby increasing the bone density in this region. Similarly, in RA superior migration of the humeral head results in load transfer to the superior glenoid vault thereby increasing the bone density in this region. Tensile stresses exerted by muscles and ligaments also influence bone mineralisation density patterns, particularly the bone beneath the supraglenoid tubercle - attachment site of the long head of biceps tendon and beneath the infraglenoid tubercle - attachment site of the long head of the triceps tendon. Exploiting this knowledge by anchoring into these regions of anticipated denser bone superiorly and posteriorly should reduce the incidence of radiolucencies by relying on stress induced osteogenic (bone-remodelling) activity to prevent the formation of a membrane of fibrous tissue at the bone-prosthesis interface.

Considering that rocking of the component in response to eccentric loads ('rocking horse' phenomenon) has been identified as a root cause of glenoid loosening anterior-posterior rocking is a concern in GHOA while superior-inferior rocking is a concern ion RA. Anchoring the component into less dense bone inferiorly and anteriorly is therefore necessary also. Fixation design should also reflect the dominant loading conditions - typically compressive superiorly and posteriorly and tensile inferiorly and anteriorly.

Eccentric loading occurs naturally in normal glenohumeral motion. In symptomatic shoulders however muscle atrophy in response to pain accentuates this peripheral loading, especially superiorly. Franklin et al.'s study clearly associates such rotator cuff muscle deficiency with glenoid loosening, confirming the role played by the 'rocking horse' phenomenon. The incidence of rotator cuff tears in the asymptomatic population alone (over 60 years old) is as high as 50%. Since the concept of the rocking horse glenoid phenomenon was introduced in the late 90's the indications for glenoid resurfacing have receded. As a result the patient suffers from limited pain relief from glenoid arthritis - the primary indication of shoulder arthroplasty and the most common reason for revision surgery among patients with hemiarthroplasty. Glenoid loosening due to these toggling forces to be tackled by improved component design.

As compliant bearings show reduced shear stress in the centre of the contact they may also provide more shock absorption to eccentric, rocking forces than the current accepted UHMWPE standard components. Compliant layer technology also provides a medium through which loads are distributed more evenly across the subchondral layer, similar to articular cartilage.

Studies of trabecular bone architecture have shown that trabeculae in the middle portion of the glenoid are radially oriented perpendicular to the subchondral plate while peripherally trabeculae are oriented towards the next nearest cortical bone i.e. the cortical base of the scapular spine (spinoglenoid notch) or the cortical axiallary border. The lateral border and the spine act as the pillars of the scapula [Anetzberger, H. and Putz, R. Morphometry of the sub-acrominal space and its clinical relevance. Unfallchirurg. 1995 Aug. 98(8):407-14.]. A more bio-mechanically compatible glenoid design would therefore aim to transfer stress to these regions of cortical bone with anchoring devices aligned with trabeculae where possible to allow near normal stress transfer to occur.

Minimal depth of bone is available for fixation anteriorly and posteriorly while the glenoid vault is deepest along the centreline running from superior to inferior.

A number of early fully constrained shoulder arthroplasty devices utilised extra long pegs directed inferiorly into the cortical axillary border of the scapula blade. Good fixation was reported to have been achieved despite early failure of all constrained devices, e.g. Stanmore, Fenlin & Trispherical.

There are three types of fixation methods.

Mechanical interlock by press fitting, using PMMA or using bone screws, biological fixation through bone in-growth and direct chemical bonding by coating with calcium hydroxyapatite. N one method of fixation has proven successful in long-term glenoid fixation therefore a combination of proven fixation mechanisms is proposed.

Mechanical interlock provides immediate stability allowing early mobility, preventing muscle atrophy and fibrous tissue growth. Those skilled in the art are already familiar with this proven fixation method reducing complexity and complications. This method is chosen for anchoring the four poles and must be secure enough to allow early passive movement.

Reaming the glenoid surface has been accepted as part of routine implantation procedure, providing maximum underlying bone support for the glenoid component, which is important to ensure natural stress transfer to the subchondral bone. As part of this procedure a 15-30mm guide hole must first be drilled into the scapula. This pilot hole an be utilised for bone in-growth to provide long-term component fixation. The location of this fixation is ideal given that micro-motion is minimal here as the component is loading predominantly peripherally creating a rocking motion about this point. Mechanical interlock fixation peripherally ensures minimal micromotion centrally providing optimal conditions for bone ingrowth. The proven reduction of radiolucencies at the component-bone interface advocates utilising bone-ingrowth fixation. Complications associated with bone-ingrowth systems include accelerated polyethylene wear due to metal backing and dissociation from the metal tray. Removing the metal backing effectively remove these complications, strengthening the probability of good long-term fixation.

The remaining component under-surface may be coated with calcium hydroxyapatite to promote chemical bonding to the surface of the natural glenoid. The backing material itself may additionally or alternatively be compounded with hydroxyapatite. This will also aid long-term fixation as well as manage high shear forces. Significantly reduced instances of radiolucent lines have been observed with the hydroxyapatite coating Copeland Mark 3 glenoid prosthesis which has been in use since 1993.

Several finite element analysis of glenoid designs found that all-polymer implants provide a more physiological stress distribution than metal back components. Walch et al. [Walch, G., Edwards, T.B., Boulahia, A., Boileau, P., Molé, D., Adeleine, P. 2002. The influence of glenohumeral prosthetic mismatch on glenoid radiolucent lines. The journal of bone and joint surgery. Dec. 2002, 84-A, No.12, pp. 2186-2192.] concluded that the incidence of loosening of metal-backed glenoids is significantly higher than that observed with polyethylene glenoids and is correlated with deteriorating functional results and increasing pain. Pegged components also provide a stress distribution in the surround bone more similar to the anatomic glenoid than keeled designs.

Keeled components have however demonstrated reduced migration following eccentric loading in the study by Anglin et al. [Anglin, C., Wyss, U.P., Nyffeler, R.W. and Gerber, C. Loosening performance of cemented glenoid prosthesis design pairs. Clinical Biomechanics. Vol. 16, Issue 2, Feb. 2001, pp.144-150.]

Lazarus et al. [Lazarus, M.D., Jensen, K.L. and Matsen, F.A. III. The Radiographic Evaluation of Keeled and Pegged Glenoid Component Insertion. J Bone Jt Surg 84-Am: 1174-1182 (2002).] determined that superior technical results are associated with pegged components. Radiolucencies and incomplete component searing occur more frequently in associated with keeled components.

FR-A-2 683 142 shows a prosthetic glenoid construction which comprises two pieces one of which is located on the scapula by screws and to which the other piece is connected. There are complications with two piece constructions as the two parts have to be held together and, moreover, the use of screws can make location and engagement of the parts on the scapula difficult especially if the screws are not at right angles to the component which they are attaching.

According to the present invention a prosthetic glenoid component for attachment to a scapula to provide a bearing for the humeral head in a shoulder prosthesis comprises a one-piece bearing element having a concave lateral bearing surface for contact with the humeral head with which it is to be used and an opposing medial surface for attachment to a scapula characterised in that the bearing element has two flexibly resilient affixation pegs can be bent resiliently towards each other to allow them to be inserted in engagement openings in the scapula with which they are to be used and which project from the medial face thereof, one of said pegs being at a superior position which projects in a superior direction of said pegs being angled in rotation to the medial-lateral direction, and the other which is located in an inferior position and which projects in an inferior direction which is angled in relation to the medial-lateral direction.

The angle between the pegs is preferably approximately 10° greater than the angle circumscribed if the pegs were perpendicular to the bearing element's curved backing.

Again, the pegs can be bent resiliently towards each other to allow them to be inserted in engagement openings in the scapula with which they are to be used.

An intermediate peg can be provided projecting from the medial face of the glenoid component which is located between the superior and inferior affixation pegs and which is dimensioned to engage a centrally located guide hole used for reaming the anatomical glenoid surface of the joint in which the glenoid component is to be used. With this construction the bearing element can again have a soft low modulus concave lateral bearing surface and an opposing medial surface which is substantially harder and carries the two affixation pegs and the bearing element can be substantially oval shaped.

With these arrangements the bearing element can again have a soft low modulus concave lateral bearing surface and an opposing medial surface which is substantially harder and carries the two affixation pegs, and in which the soft low modulus bearing surface extends around the periphery of the bearing element and increases its thickness to provide a deformable rim to simulate the labrum in a anatomical glenoid.

Preferably in the bearing surface can be made from a soft elastomeric polyurethane material.

The soft elastomeric polyurethane material has a hardness value of 3.0 to 9.0 N/mm² using hardness testing method BS 2782; Pt 13 method 365D.

Such a soft elastomeric polyurethane material is Bionate 80A.

The medial surface in any of the constructions can be made from a rigid polymeric material and such a material can have a minimum hardness value of 65 N/mm² using hardness testing method BS 2782; Pt 13 method 365D.

Thus, the rigid polymeric material is preferably polyurethane, for example, Bionate 75D.

If desired the medial surface can be provided on a backing portion at least part of which is made from a porous trabecular metal structure.

The invention can be performed in various ways and some embodiments will now be described by way of example and with reference to the accompanying drawings in which :
Figure 1 is a cross-sectional diagrammatic view of a glenoid component showing radial mismatch and its contribution to BSA;
Figure 2 is a diagrammatic cross-sectional view of a prosthetic glenoid component showing variations in glenoid width and its contribution to BSA and which, when combined with construction shown in Figure 1, shows the total balance stability angle (BSA) in antero-posterior direction;
Figure 3 is a diagrammatic isometric view of a prosthetic glenoid component according to the invention from one side;
Figure 4 is a diagrammatic isometric view of the construction shown in Figure 1 taken from one side and below;

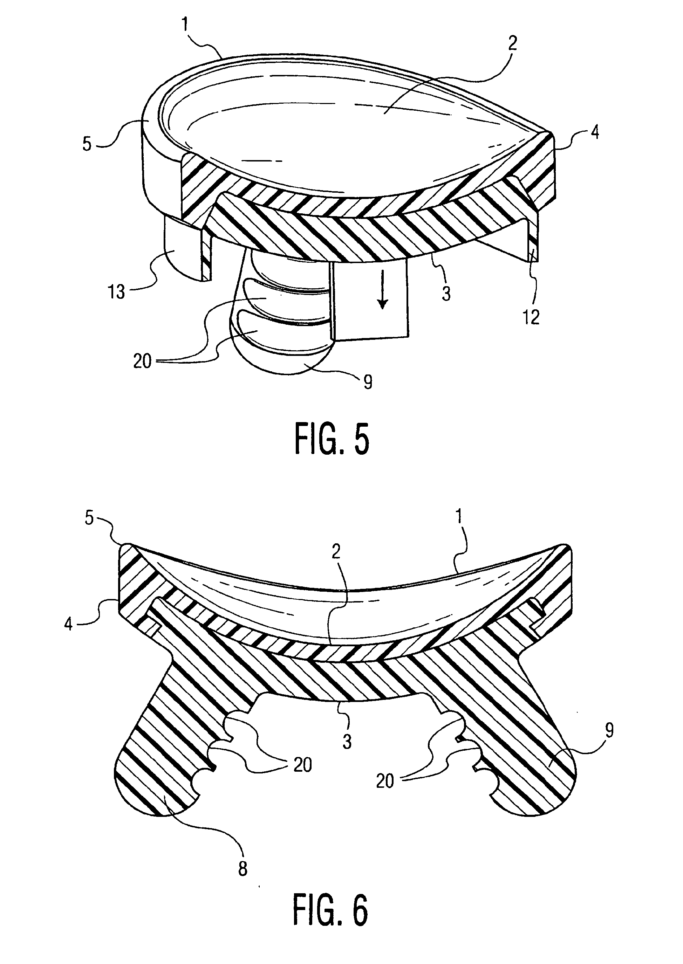

Figure 5 is a diagrammatic isometric view of the construction shown in Figures 1 and 2 taken in partial cross-section from one end and above;
Figure 6 is a cross-sectional side elevation of the construction shown in Figures 3 to 5;
Figure 7 is a diagrammatic view showing how the glenoid component shown in Figures 2 to 6 is fitted into place in the natural glenoid;
Figures 8 to 10 are isometric views of a drill guide for use in the invention;
Figures 11 and 12 are further view showing how the glenoid component is fitted in place in the natural scapula;
Figures 13, 14 and 15 are respectively a plan view, end elevation and isometric view from one end showing the glenoid element according to the present invention in place;
Figure 17 is a diagrammatic cross-sectional view of a glenoid component which has a backing portion and each part of which is made from a porous trabecular metal structure;
Figure 18 shows another construction similar to Figure 18 in which the trabecular metal structure is incorporated into part of the central peg;
Figure 19 shows a central peg which is formed as a porous trabecular metal structure;
Figure 20 shows how the two affixation pegs can be formed as porous trabecular metal structures;
Figures 21, 22 and 23 are part cross-sectional diagrammatic side elevations showing further peg constructions which employ porous trabecular metal structures;
Figures 24, 25 and 26 are cross-sectional diagrammatic plan views of the structure shown in Figures 21, 22 and 23; and,
Figure 27 is a diagrammatic cross-sectional view of a glenoid component which has a backing portion which is entirely formed as a porous trabecular metal structure.

As shown in the drawings a prosthetic glenoid component according to the present invention for attachment to a scapula to provide a bearing for the humeral head in a shoulder prosthesis comprises a substantially oval shaped one-piece bearing element 1 having a soft low modulus concave lateral bearing surface 2 for contact with the humeral head with which it is to be used and an opposing medial surface 3 which is substantially harder for attachment to a scapula.

The soft low modulus concave lateral bearing surface 2 is provided by a soft polyurethane layer which is bonded to a harder polyurethane backing material which provides the surface 3. The invention provides superior lubrication by using the soft PU as a bearing surface with the humeral head. This design feature resolves the contradictory requirements of radial mismatch common to other glenoid designs.

The soft bearing surface 2 extends around the periphery 4 of the bearing element 1 and increases its thickness to provide a deformable rim 5 to simulate the labrum in an anatomical glenoid.

The first advantage of this is it allows the balance stability angle (BSA) to be substantially increased, effectively deepening the socket, similar to the labrum in the biological glenoid. The risk of impingement of the humerus on the glenoid rim remains, however the soft polyurethane layer will not undergo permanent deformation therefore the components contribution to stability is maintained. The soft layer provides shock absorption to impingement thereby minimising the shear stress transfer to anchoring features. Earlier glenoid designs have suffered from sheared screws and pegs as well as disengagement of polyethylene bearing surfaces as a result of these shear forces. Bevelling of the glenoid rim to reduce impingement, as carried out in some glenoid designs, minimises available articular surface and predisposes to instability by reducing the balance stability angle. Again the designer is forced into a trade off between component failure through impingement induced loosening or through instability. The second advantage of wrapping around the periphery prevents lift off of the soft polyurethane bearing layer from the harder polyurethane backing.

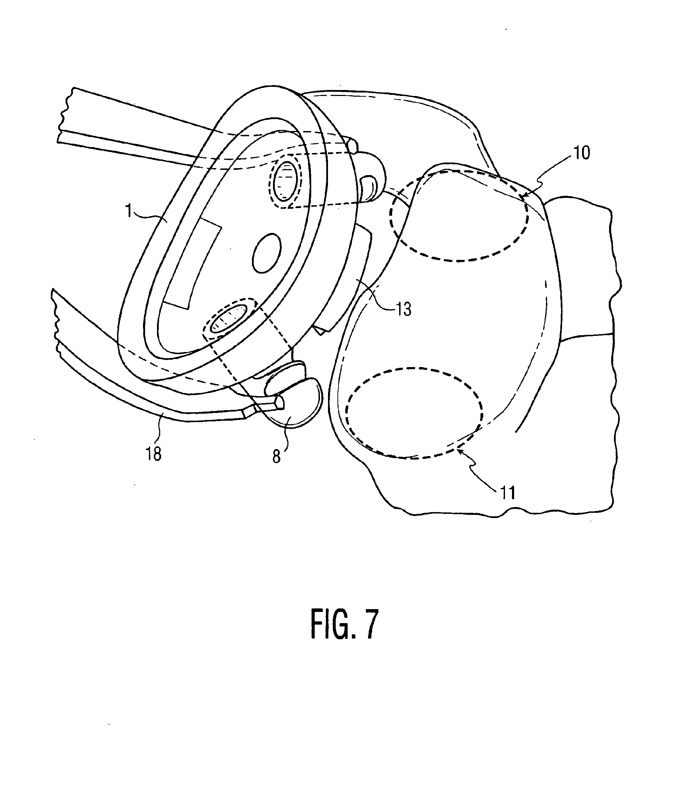

Those skilled in the art will appreciate that the glenoid component can be made in a variety of sizes for use with large and small patients. Based on studies of glenoid size and shape [Checroun, A.J., Hawkins, C., Kummer, F.J., Zuckerman, J.D. Fit of current glenoid component designs: an anatomic cadaver study. Journal of shoulder and elbow surgery. 2002 Vol. II, No.6, pp. 614-617.] the invention can include a substantially oval-shaped bearing element 1 which is pear-shaped with relative dimensions of 1 in the anterior-posterior direction and 1.3 in the superior-inferior direction. As illustrated in Figure 7 this design of the component footprint allows a better fit with the natural glenoid therefore maximising underlying bone support for the hard polyurethane backing and minimising overhand which can result in large rocking moments across the prosthesis. Additionally, while not yet proven, the teardrop shape of the glenoid cavity, being wider inferiorly than superiorly, must also play a function in joint kinematics, perhaps ensuring full internal or external rotation without inpingement of humeral metaphysis. Similarly Hertel and Ballmer [Hertel, R., Ballmer, F.T. 2003. Observations on retrieved glenoid components. J Arthroplasty. Vol.18, No.3, 2003, pp.361-366.] highlight that available glenoid components may cover an excessive amount of the head resulting in abutment against the glenoid rim.

Articular cartilage varies from 2-4 mm in thickness in the large joints of adults. [Unsworth, A., Roberts, B., and Thompson, J.C. The application of soft layer lubrication to hip prosthesis. J Bone Jt Surg., 1981, 63-B, 297. Unsworth, A., Pearcy, M.J., White, E.F.T., and White, G. Soft layer lubrication of artificial hip with reduced wear rates, joints. Proceedings of the International Conference on tribology, friction, lubrication, and wear, 50 years on, 1987, Mechanical Engineering Publications Ltd., London, 715-724.

Unsworth, A., Pearcy, M.J., White, E.F.T., and White, G. Frictional properties of artificial hip joints. J. Engng in Medicine, 1988, 17(3), 101-104. Weldon, E.J., Scarlat, M.M., Lee, S.B., and Matsen, F.A. Intrinsic stability of unused and retrieved polyethylene glenoid components. J. Shoulder Elbow Surg. 2001, 10(5), 474-481.] Allowing for 1-2 mm additional depth following glenoid reaming and no cement between reamed surface and the component backing, ideal glenoid thickness should range from 3-6 mm. Consideration will be made for the fact that thicker glenoid components may help manage contact stresses. However, the importance of reconstructing the lateral humeral offset to within 2-3 mm and avoid "stuffing" the joint must also be considered. Published data on compliant layer hip prostheses indicates that the decrease in contact pressure and shear stress with an increase of compliant layer thickness becomes less pronounced for thicknesses greater than 2 mm. Compliant layer thickness will therefore preferably be 1-2 mm thick with a stiffer backing thickness of 2-4 mm.

With the introduction of a compliant bearing surface the requirement for radial mismatch between the numeral head and glenoid component is reduced. Radial mismatch is minimised to provide maximum contact area and minimal contact stress. Radial mismatch will account for deformation of the soft layer while maintaining optimal lubrication conditions. The range of radial mismatch will lie between those of the anatomical joint and the standard UHMWPE replacement, i.e. 0.1-2 mm radial mismatch. Flaring the glenoid rim may also be required to prevent pinching of the humeral head and lubricant starvation after excessive creep of the polyurethane.

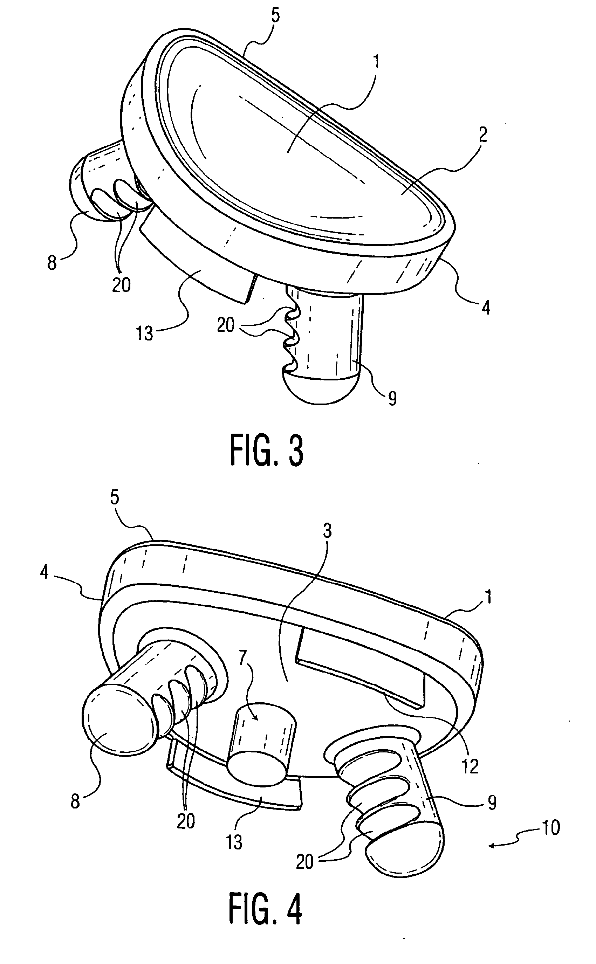

The element 1 also has an intermediate or central peg 7, most clearly shown in Figure 4. This peg can have cavities moulded into it (not shown) for receiving bone graft from the resected humeral head, allowing a good fit to be obtained without precise bone preparation. The peg 7 utilizes the necessary guide hole which has been previously drilled in the bone. Guide holes of this kind are also used for reaming the anatomical glenoid surface of the joint with which a glenoid component is to be used.

The peg surface will have characteristics of porosity, pore size and depth compatible with both fibrous tissue and bone ingrowth. This fixation device is intended to reinforce long-term component purchase in glenoid bone; as such it is a secondary fixation method, which is not critical to fixation performance. In rheumatoid arthritis the bone quality may dictate the use of bone cement with this central peg.

Fixation design is based on the principle of anchoring into good bone where possible or deep into poorer bone, again where possible. Denser bone indicates greater levels of bone remodelling activity. Anchoring into this bone ensures greater possibility of maintaining long term fixation due to the minimal bone resorption as indicated by fibrous tissue growth or radiographic lucencies.

According to the present invention the element 1 also has two affixation pegs 8 and 9 which project from the medial face 3, peg 8 being at a superior position and which projects in a superior direction which is angled in relation to the medial-lateral direction and the peg 9 being located in an inferior position and projecting in an inferior direction which is angled in relation to the medial-lateral direction. These divergent pegs 8 and 9 are intended to be anchored into the greater glenoid vault both superiorly, ad indicated by the area 10 in Figure 7 and inferiorly as indicated by the area 11, and at a maximum distance apart are intended to prevent superior-inferior rocking horse forces.

The orientation of the superior and inferior divergent pegs coupled with the compliant nature of the polyurethane material enables the pegs 8 and 9 to be resiliently bent towards each other so that the component can be a snap fit into place. The angle between the pegs is proposed to be approximately 10° greater than the angle circumscribed if the pegs were perpendicular to the curved component backing. This ensures a mechanical interlock while closely aligning the pegs with the cancellous trabeculae.

Thus the pegs can be angled at approximately 30 ° to the medial-lateral direction.

The medial surface of the element 1 is also provided with a pair of projecting flanges 12, 13 located at or adjacent the anterior and posterior rim.

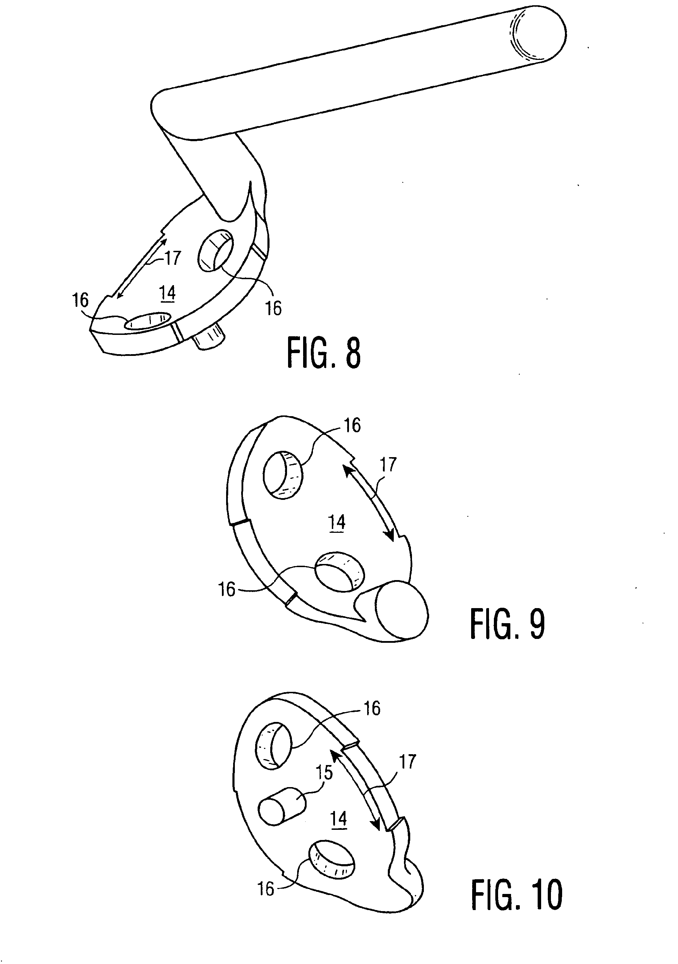

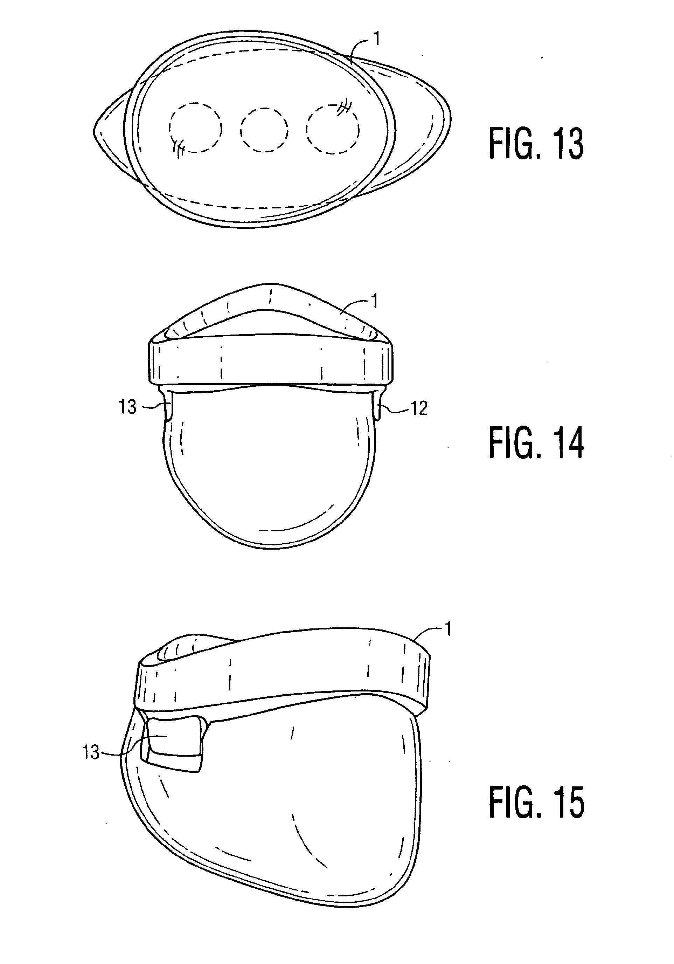

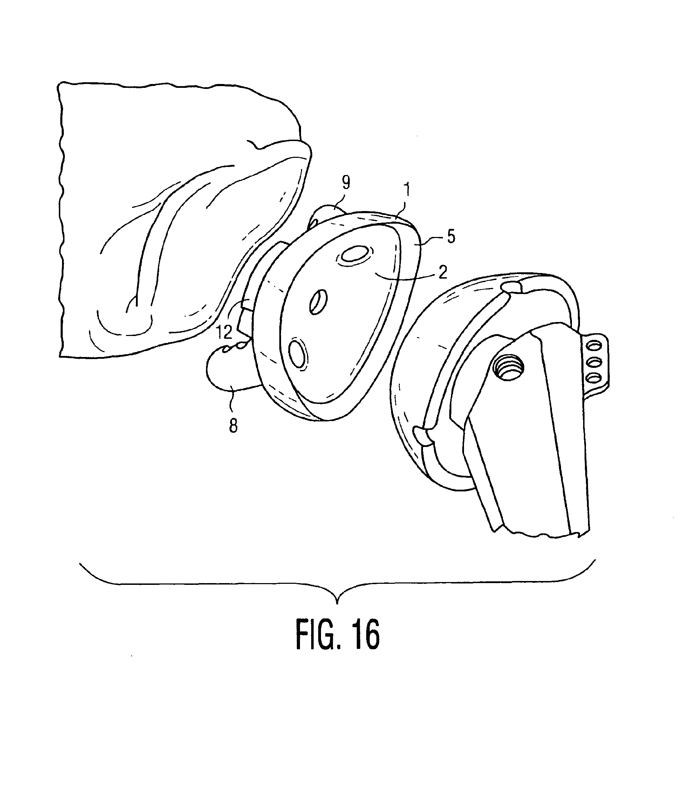

The method of fitting the glenoid component is similar to the current standard in glenoid implanting procedure, the capsule is released, all osteophytes on the glenoid rim are removed and a guide or centring hole is drilled. A spherically cut face is developed centrally using a glenoid reamer and guide hole. A drill guide 14, as shown in Figures 8 to 10, has a guide boss 15 which is placed in the pre-drilled hole and the 60 ° divergent holes are accurately drilled in to the glenoid cavity using guide opening 16. The anterior and posterior glenoid rim is then prepared as in Figure 15 to receive the short flanges 12, 13 on the component using a high speed burr along the guide in the position indicated by reference numeral 17.

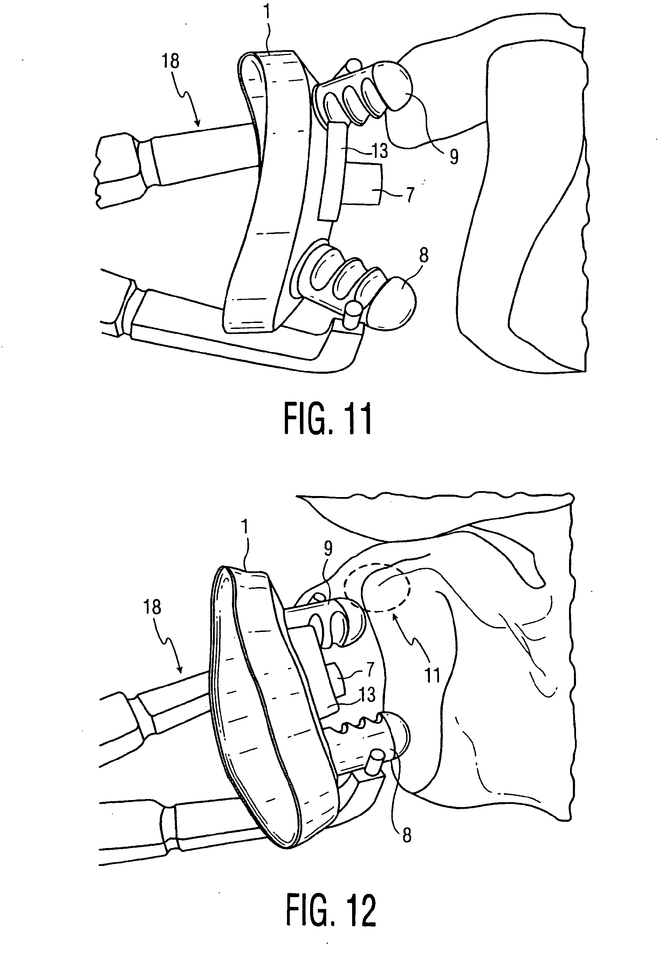

The component is incubated at 37°C prior to implanting, as shown in Figures 11 and 12. A special clamping device 18 is used to flex the pegs 8 and 9 (Approx. 250 Newtons) and locate them in their prepared holes. Once located a glenoid pusher is used to gently push the component home after removing the clamp 18 and allowing the pegs 8 and 9 to extend. This method allows ease of implantation while preventing damage to the compliant bearing surface 2. Grooves 20 designed into the pegs 8 and 9 allow for easier flexion and act as macro structures for bone cement interdigitation if the use of bone cement is preferred. The circular cross section of the pegs assures an appropriate fit with ease of preparation and reduction of stresses.

According to another aspect of the invention the superiorly directed peg 9 anchors into the bone beneath the supraglenoid tubercle, indicated by area 21 in Figure 12, the long head of biceps attachment site. It is known that as rotator cuff tears progress the biceps muscle is recruited even more as a joint stabiliser. As such, greater activity of this tendon will initiate greater levels of bone remodelling or osteogenic activity ensuring good long-term purchase of the superiorly directed peg. The tubercle can be situation anywhere from 11-1 o'clock however and the site of tendon origin itself is somewhat variable.

The short flanges 12 and 13 located at the anterior and posterior rim help prevent transfer of impingement induced shear forces to centrally located anchoring devices.

Maximising the area of fixation between natural bone and prosthetic component through hydroxyapatite coating of the entire backing will also contribute to controlling high shear forces. The convex backing 3 also helps with eccentric loading induced shear forces.

The three anchoring pegs 7, 8, 9 as well as the overhanging flanges 12, 13 prevent rotational shear forces which may occur.

Based on the design charts published by Yao [Yao, J.Q., Parry, T.V., Unsworth, A., Cunningham, J.L. 1994. Contact mechanics of soft layer artificial hip joints. Part 2: application to joint design. Proc Instn Mech Engrs. Vol. 208 Part H, pp. 206-215.] the contact radius and max shear stress encompassing the complete range of design parameters is 10 - 20 mm and 2.0.5 Mpa respectively. Yao's range [Yao, J.Q., Parry, T.V., Unsworth, A., Cunningham, J.L. 1994. Contact mechanics of soft layer artificial hip joints. Part 2: application to joint design. Proc Instn Mech Engrs. Vol. 208 Part H, pp. 206-215.] calculations were with particular reference to compliant layer hips where a 3 kN load (around 4 times body weight) was investigated. This load is 4 times greater than those expected during activities of daily living involving the shoulder joint and so represent very conservative values.

The most recent publication on the use of compliant bearing technology by Scholes et al. [Scholes, S.C., Unsworth, A., Blamey, J.M., Burgess, I.C., Jones, E. and Smith, N. Design aspects of compliant, soft layer bearings for an experimental hip prosthesis Proceedings Of The Institution Of Mechanical Engineers. Part, H., J Engng in Medicine, Vol. 219, Issue 2, 2005, pp. 79-87.] indicates an optimal compliant layer hardness of 4 - 6 Nmm⁻² Bionate 80A (5.56 Nmm⁻²) is chosen for early prototypes as it has a modulus of elasticity value very similar to that of articular cartilage (6-10 Mpa) and has proven biocompatibility. Provision will be made however to minimise permanent deformation / set as well as shear strains possibly by blending with appropriate material or addition of fibre composite to both the softer bearing surface and the harder backing material.

In the constructions described above the two materials used are the soft PU for the bearing function and a hard PU (or a composite material thereof) for the support material.

A porous trabecular metal structure can also be used to enable bone to grow into the central peg 7 or into a portion of the angular orientated pegs 8 or 9. The porous trabecular metal structure can also be used to enable bone growth at any part of a backing portion.

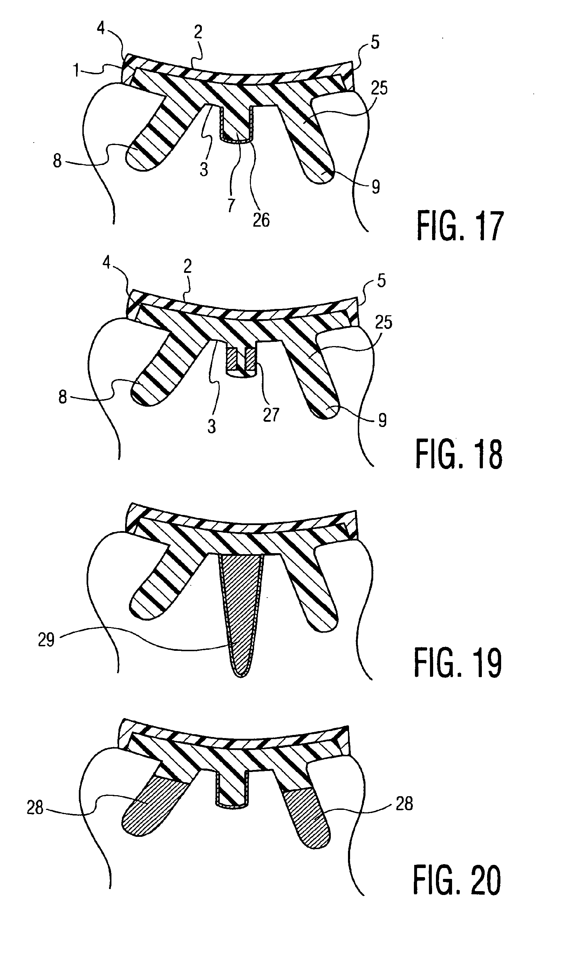

Figures 17 to 27 illustrate the above features and the same reference numerals are used to indicate similar parts to the previous Figures.

As shown in Figure 17 the glenoid component comprises a substantially oval-shaped one-piece bearing element 1 which has a surface 2 and the opposing medial surface 3 is formed on a backing member 25 on which the pegs 7,8 and 9 are provided. The overhanging flanges 12, 13 are also provided but are not shown in these Figures. The pegs can be made from, for example, by selective layers of sintering and can be incorporated into the mould during the injection moulding process involving the formation of the hard PU substrate. As shown in Figure 17 the central peg can be entirely bounded by the porous metal (as indicated by reference numeral 26) on its outer surface or, as shown in Figure 18, could be applied as a band 27 so that it only partially covers the peg.

If such a porous covering is applied to the angularly orientated pegs 8 and 9 it is only applied to the lower portion, as indicated in Figure 20 by the reference numeral 28 so that it will allow the pegs to be deflected when surgically implanted. The construction could be such that the trabecular metal structure would merely surround part of the pegs but could provide the lower ends of the pegs themselves.

Figure 19 shows a construction in which the entire central peg 7 is made as a porous trabecular metal structure indicated by reference numeral 29.

A third design of peg could have layers of form of 1-1.5 mm in thickness and be of porous trabecular metal an inner core being of open lattice which is able to interlock with the hard PU at the contact points. A dense or semi-permeable wall could be incorporated to separate the trabecular structure from the open lattice to prevent hard PU from entering the trabecular region during the moulding process. This design gives the benefits of bone interlock and a more stable structure, with the same level of sub-condral bone being maintained.

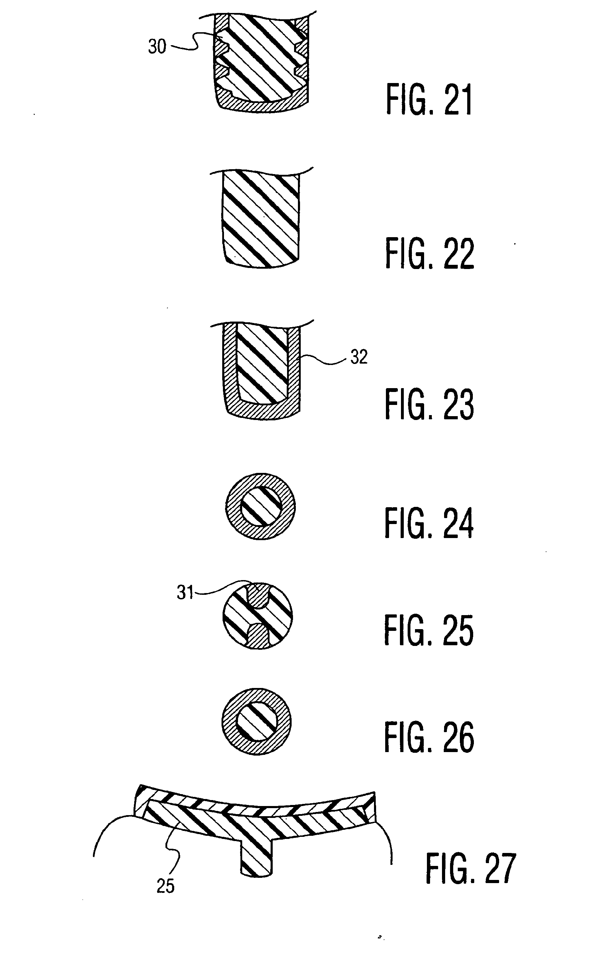

Figures 21 and 24 show an arrangement in which the porous trabecular metal structure is provided as a skin with interlocking inner grooves 30.

Figures 22 and 25 shows how the porous trabecular metal structure could be provided as inserts which are indicated by reference numeral 31 in Figure 25 and Figures 23 and 26 show how the porous trabecular metal structure could be provided as a skin 32 which surrounds a peg.

If the preservation of sub-condral bone is not a major requirement then it is possible to provide a backing member 25, as shown in Figure 27, in which the porous metal replaces the hard PU.

The porous trabecular metal structure, when used with the hard PU, can have a polymer engaging portion, an intermediate portion and a bone ingrowth portion. The polymer engaging portion can be made up of various structures, walls, beams and appendages so as to form a matrix-like lattice having a porosity. The porosity of the polymer engaging portion is chosen so as to best aid in the incorporation of the PU. A preferred pore size may be approximately between 500 microns to 1,200 microns, the pore size will depend upon the requirements.

The polymer engaging portion is preferably made of a bio-compatible material such as, but not limited to, titanium or a similar metal structure.

The intermediate portion disposed between the polymer engaging portion and the bone ingrowth portion preferably has a porosity that is significantly less than the porosity of the polymer engaging portion and is low enough such that the polymer body when being attached to the polymer engaging portion the polymer material is unable to come into contact with the bone ingrowth portion. Thus the intermediate portion acts as a barracade in preventing any leaching of the polymer material into the bone ingrowth portion.

The bone ingrowth portion may be similarly constructed to the polymer engaging portion and constructed with a particular porosity so that when the implant is implanted in the bone the bone growth portion will promote bone ingrowth by the surrounding tissue.

Methods of making porous metal structures are shown in US Patent Applications Nos. 10/704,270 entitled, "Laser-Produced Porous Surface"; 11/027,421 entitled, "Gradient Porous Implant"; 11/295,008 entitled, "Laser-Produced Porous Surface"; and 60?755,260 entitled, "Laser-Produced Implants". As discussed in US Patent Application No. 10/704,270, the metal structure may be constructed using a selective laser melting or sintering process, which hereby grows the structure in a layer by layer process. In the alternative, the metal structure may be built using an alternate process described in US Patent Application No. 10/704,270 wherein the intermediate portion acts as a base or substrate on which the polymer engaging portion and bone ingrowth portion are built thereon, also in a layer-by-layer fashion. Additional techniques for constructing the metal lattice may also be employed such as that disclosed in US Patent Application No. 10/071,667 entitled, "Porous Metallic Scaffold for Tissue", as well as additional methods known to those in the art such as that disclosed in Patent Corporation Treaty Application 2005/023118 entitled "Porous Metal Articles Formed Using an Extractable Particulate," filed on July 22, 2004.

## Claims

1. A prosthetic glenoid component for attachment to a scapula to provide a bearing for a humeral head in a shoulder prosthesis which comprises a one-piece bearing element (1) having a concave lateral bearing surface (2) for contact with the humeral head with which it is to be used and an opposing medial surface (3) for attachment to a scapula **characterised in that** the bearing element (1) has two flexibly resilient affixation pegs (8)(9) can be bent resiliently towards each other to allow them to be inserted in engagement openings in the scapula with which they are to be used and which project from the medial face (3) thereof, one of said pegs (8)(9) being at a superior position which projects in a superior direction of said pegs (8)(9) being angled in relation to the medial-lateral direction, and the other which is located in an inferior position and which projects in an inferior direction which is angled in relation to the medial-lateral direction.

2. A prosthetic glenoid component as claimed in claim 1 in which the angle between the pegs (8)(9) is approximately 10° greater than the angle circumscribed if the pegs were perpendicular to the bearing element's (1) curved backing.

3. A prosthetic glenoid component as claimed in claim 1 or claim 2 **characterised by** including an intermediate peg (7) projecting from the medial surface (3) of the glenoid component (1) which is located between the superior and inferior affixation pegs (8)(9) and which is dimensioned to engage a centrally located guide hole used for reaming the anatomical glenoid surface of the joint in which the glenoid component is to be used.

4. A prosthetic glenoid component as claimed in any one of claims 1 to 3 **characterised in that** the bearing element (1) has a soft low modulus concave lateral bearing surface (2) and an opposing medial surface )3) which is substantially harder and carries the two affixation pegs (8)(9).

5. A prosthetic glenoid component as claimed in claim **characterised in that** the bearing element (1) is substantially oval shaped.

6. A prosthetic glenoid component as claimed in any one of preceding claims 1 to 5 **characterised in that** the bearing element (1) has a soft low modulus concave lateral bearing surface (2) and an opposing medial surface (3) which is substantially harder and carries the two affixation pegs (8)(9), and in which the soft low modulus bearing surface (2) extends around the periphery of the bearing element (1) and increases its thickness to provide a deformable rim (5) to simulate the labrum in a anatomical glenoid.

7. A prosthetic glenoid component as claimed in any one of the preceding claims **characterised in that** the bearing surface (2) is made from a soft elastomeric polyurethane material.

8. A prosthetic glenoid component as claimed in claim 7 **characterised in that** the soft elastomeric polyurethane material has a hardness value of 3.0 to 9.0 N/mm² using hardness testing method BS 2782; Pt 13 method 365D.

9. A prosthetic glenoid component as claimed in claim 7 or claim 8 **characterised in that** the soft elastomeric polyurethane material is Bionate 80A.

10. A prosthetic glenoid component as claimed in any one of the preceding claims **characterised in that** the medial surface is made from a rigid polymeric material.

11. A prosthetic glenoid component as claimed in claim 10 **characterised in that** the rigid polymeric material is polyurethane.

12. A prosthetic glenoid component as claimed in claim 11 **characterised in that** the rigid polyurethane material has a minimum hardness value of 65 N/mm² using hardness testing method BS 2782; Pt 13 method 365D.

13. A prosthetic glenoid component as claimed in claim 12 **characterised in that** said polyurethane is Bionate 75D.

14. A prosthetic glenoid component as claimed in any one of the preceding claims **characterised in that** the medial surface (3) is provided with a pair of projecting flanges (12)(13) located at or adjacent to the anterior and posterior rim.

15. A prosthetic glenoid component as claimed in any one of the preceding claims **characterised in that** said medial surface (3) is provided on a backing portion (25) at least part of which is made from a porous trabecular metal structure.

16. A prosthetic glenoid component as claimed in claim 15 **characterised in that** substantially the whole of the backing portion (25) is made as a porous trabecular metal structure.

17. A prosthetic glenoid component as claimed in claim 15 **characterised in that** the backing portion (25) is made from PV with porous trabecular metal structure portions.

18. A prosthetic glenoid component as claimed in any one of claims 15, 16 or 17 **characterised in that** the porous trabecular metal structure is made by depositing a first layer of a powder made from a metal selected from the group consisting of titanium, titanium alloys, stainless steel, cobalt chrome alloys, tantalum and niobium, onto a substrate; scanning a laser beam at least once over said first layer of powder, said laser beam having a power (P) in Joule per sec. with a scanning speed (v) in millimetres per sec., and a beam overlap (b) in millimetres such that the number calculated by the formula P/(b x v) lies between the range 0.3-8 J / mm², said beam overlap being approximately between 0% to - 1200% to give the required pore size; depositing at least one layer of said powder onto said first layer; and repeating said laser scanning steps for each successive layer until a desired web height is reached.

19. A prosthetic glenoid component as claimed in any one of claims 15, 16 or 17 **characterised in that** the porous trabecular metal structure is made by depositing a first layer of a powder made from a metal selected from the group consisting of titanium, titanium alloys, stainless steel, cobalt chrome alloys, tantalum and niobium, onto a substrate; and scanning a laser beam having a power (P) for a period of time (µsec) with a point
distance (µm), to form a portion of a plurality of predetermined unit cells within said metal powder.

## Patentansprüche

1. Prothetische Glenoidkomponente zur Befestigung an einem Schulterblatt, um in einer Schulterprothese ein Lager für einen humeralen Kopf bereitzustellen, umfassend ein einstückiges Lagerelement (1) mit einer konkaven lateralen Lageroberfläche (2) zum Kontakt mit dem humeralen Kopf, mit dem sie verwendet werden soll, und einer entgegengesetzten medialen Oberfläche (3) zur Befestigung an einem Schulterblatt, **dadurch gekennzeichnet, dass** das Lagerelement (1) zwei nachgiebig elastische Befestigungszapfen (8)(9) aufweist, die elastisch aufeinander zu gebogen werden können, um es zu gestatten, sie in Eingriffsöffnungen in dem Schulterblatt einzusetzen, mit dem sie verwendet werden sollen, und die aus seiner medialen Seite (3) überstehen, wobei sich einer der Zapfen (8)(9) an einer oberen Position befindet, welcher in einer oberen Richtung der Zapfen (8)(9) übersteht, die in Bezug zur medial-lateralen Richtung angewinkelt ist, und von denen der andere in einer unteren Position angeordnet ist, und welcher in einer unteren Richtung übersteht, die in Bezug zur medial-lateralen Richtung angewinkelt ist.

2. Prothetische Glenoidkomponente nach Anspruch 1, bei welcher der Winkel zwischen den Zapfen (8)(9) ungefähr 10° größer als der Winkel ist, der umschrieben würde, wenn die Zapfen senkrecht zu der gekrümmten Unterlage des Lagerelements (1) wären.

3. Prothetische Glenoidkomponente nach Anspruch 1 oder Anspruch 2, **dadurch gekennzeichnet, dass** sie einen aus der medialen Oberfläche (3) der Glenoidkomponente (1) überstehenden intermediären Zapfen (7) einschließt, der zwischen dem oberen und dem unteren Befestigungszapfen (8)(9) angeordnet ist und der bemessen ist, um in eine mittig angeordnete Führungsöffnung einzugreifen, die zum Räumen der anatomischen Glenoidoberfläche des Gelenks, in dem die Glenoidkomponente verwendet werden soll, benutzt wird.

4. Prothetische Glenoidkomponente nach einem der Ansprüche 1 bis 3, **dadurch gekennzeichnet, dass** das Lagerelement (1) eine weiche konkave laterale Lageroberfläche (2) mit niedrigem Modul und eine entgegengesetzte mediale Oberfläche (3) aufweist, die wesentlich härter ist und die zwei Befestigungszapfen (8)(9) trägt.

5. Prothetische Glenoidkomponente nach Anspruch, **dadurch gekennzeichnet, dass** das Lagerelement (1) im Wesentlichen oval geformt ist.

6. Prothetische Glenoidkomponente nach einem der vorangehenden Ansprüche 1 bis 5, **dadurch gekennzeichnet, dass** das Lagerelement (1) eine weiche konkave laterale Lageroberfläche (2) mit niedrigem Modul und eine entgegengesetzte mediale Oberfläche (3) aufweist, die wesentlich härter ist und die zwei Befestigungszapfen (8)(9) trägt, und bei der sich die weiche Lageroberfläche (2) mit niedrigem Modul um den Umfang des Lagerelements (1) herum erstreckt und in ihrer Dicke zunimmt, um einen verformbaren Rand (5) bereitzustellen, um das Labrum in einem anatomischen Glenoid zu simulieren.

7. Prothetische Glenoidkomponente nach einem der vorangehenden Ansprüche, **dadurch gekennzeichnet, dass** die Lageroberfläche (2) aus einem weichen elastomeren Polyurethanmaterial besteht.

8. Prothetische Glenoidkomponente nach Anspruch 7, **dadurch gekennzeichnet, dass** das weiche elastomere Polyurethanmaterial einen Härtewert von 3,0 bis 9,0 N/mm² aufweist, wenn das Härteprüfverfahren BS 2782; Pt 13 Verfahren 365D verwendet wird.

9. Prothetische Glenoidkomponente nach Anspruch 7 oder Anspruch 8, **dadurch gekennzeichnet**, das das weiche elastomere Polyurethanmaterial Bionate 80A ist.

10. Prothetische Glenoidkomponente nach einem der vorangehenden Ansprüche, **dadurch gekennzeichnet, dass** die mediale Oberfläche aus einem starren Polymermaterial besteht.

11. Prothetische Glenoidkomponente nach Anspruch 10, **dadurch gekennzeichnet, dass** das starre Polymermaterial Polyurethan ist.

12. Prothetische Glenoidkomponente nach Anspruch 11, **dadurch gekennzeichnet, dass** das starre Polyurethanmaterial einen Mindesthärtewert von 65 N/mm² aufweist, wenn das Härteprüfverfahren BS 2782; Pt 13 Verfahren 365D verwendet wird.

13. Prothetische Glenoidkomponente nach Anspruch 12, **dadurch gekennzeichnet, dass** das Polyurethan Bionate 75D ist.

14. Prothetische Glenoidkomponente nach einem der vorangehenden Ansprüche, **dadurch gekennzeichnet, dass** die mediale Oberfläche (3) mit einem Paar von überstehenden Flanschen (12)(13) versehen ist, die an oder benachbart zu dem vorderen und hinteren Rand angeordnet sind.

15. Prothetische Glenoidkomponente nach einem der vorangehenden Ansprüche, **dadurch gekennzeichnet, dass** die mediale Oberfläche (3) an einem Unterlageteil (25) vorgesehen ist, von dem zumindest ein Teil aus einer porösen trabekulären Metallstruktur besteht.

16. Prothetische Glenoidkomponente nach Anspruch 15, **dadurch gekennzeichnet, dass** im Wesentlichen die Gesamtheit des Unterlageteils (25) als poröse trabekuläre Metallstruktur hergestellt ist.

17. Prothetische Glenoidkomponente nach Anspruch 15, **dadurch gekennzeichnet, dass** der Unterlageteil (25) aus PV mit porösen trabekulären Metallstrukturteilen hergestellt ist.

18. Prothetische Glenoidkomponente nach einem der Ansprüche 15, 16 oder 17, **dadurch gekennzeichnet, dass** die poröse trabekuläre Metallstruktur hergestellt wird, indem auf einem Substrat eine erste Schicht aus einem Pulver abgelagert wird, das aus einem Metall besteht, welches aus der Gruppe bestehend aus Titan, Titanlegierungen, nicht-rostender Stahl, Kobalt-Chrom-Legierungen, Tantal und Niob ausgewählt ist; Bewegen eines Laserstrahls unter Abtastung mindestens einmal über die erste Pulverschicht, wobei der Laserstrahl eine Leistung (P) in Joule pro Sek. mit einer Abtastbewegungsgeschwindigkeit (v) in Millimetern pro Sek. sowie eine Strahlüberlappung (•) in Millimetern aufweist, so dass die durch die Formel P/(• x v) berechnete Zahl zwischen dem Bereich 0,3-8 J/mm² liegt, wobei die Strahlüberlappung ungefähr zwischen 0 % bis - 1200 % liegt, damit sich die erforderliche Porengröße ergibt; Ablagern von mindestens einer Schicht des Pulvers auf der ersten Schicht; und Wiederholen der Laserabtastbewegungsschritte für jede nachfolgende Schicht, bis eine gewünschte Steghöhe erreicht ist.

19. Prothetische Glenoidkomponente nach einem der Ansprüche 15, 16 oder 17, **dadurch gekennzeichnet, dass** die poröse trabekuläre Metallstruktur hergestellt wird, indem auf einem Substrat eine erste Schicht aus einem Pulver abgelagert wird, das aus einem Metall besteht, welches aus der Gruppe bestehend aus Titan, Titanlegierungen, nicht-rostender Stahl, Kobalt-Chrom-Legierungen, Tantal und Niob ausgewählt ist; und Abtasten mit einem Laserstrahl mit einer Leistung (P) über einen Zeitraum (µs) mit einem Punktabstand (µm), um einen Teil von einer Mehrzahl von vorbestimmten Einheitszellen innerhalb des Metallpulvers zu bilden.

## Revendications

1. Composant glénoïde prosthétique pour fixation à une omoplate pour fournir un plateau pour une tête humérale dans une prothèse d'épaule qui comprend un élément de plateau (1) en un morceau ayant une surface d'appui latérale concave (2) pour contact avec la tête humérale avec laquelle il doit être utilisé et une surface médiale opposée (3) pour fixation à une omoplate, **caractérisé en ce que** l'élément de plateau (1) a deux chevilles de fixation (8) (9) résilientes de manière flexible qui peuvent être courbées de manière résiliente l'une vers l'autre pour leur permettre d'être insérées dans des ouvertures de mise en contact dans l'omoplate dans laquelle elles doivent être utilisées et qui se projettent depuis sa face médiane (3), l'une desdites chevilles (8) (9) étant en une position supérieure qui se projette dans une direction supérieure desdites chevilles (8) (9) étant en angle relativement à la direction médio-latérale, et l'autre étant située dans une position inférieure et se projetant dans une direction inférieure qui est en angle relativement à la direction médio-latérale.

2. Composant glénoïde prosthétique selon la revendication 1 dans lequel l'angle formé entre les chevilles (8) (9) est supérieur d'environ 10 ° à l'angle circonscrit si les chevilles étaient perpendiculaires au support incurvé de l'élément de plateau (1).

3. Composant glénoïde prosthétique selon la revendication 1 ou selon la revendication 2 **caractérisé en ce qu'**il inclut une cheville intermédiaire (7) se projetant depuis la surface médiane (3) du composant glénoïde (1) qui se trouve entre les chevilles de fixation supérieure et inférieure (8) (9) et qui est dimensionnée de manière à engager un orifice de guidage centré utilisé pour aléser la surface glénoïde anatomique de l'articulation dans laquelle le composant glénoïde doit être utilisé.

4. Composant glénoïde prosthétique selon l'une quelconque des revendications 1 à 3 **caractérisé en ce que** l'élément de plateau (1) a une surface d'appui (2) latérale concave souple à faible module et une surface médiane opposée (3) qui est sensiblement plus dure et porte les deux chevilles de fixation (8) (9).

5. Composant glénoïde prosthétique selon la revendication **caractérisé en ce que** l'élément de plateau (1) est de forme sensiblement ovale.

6. Composant glénoïde prosthétique selon l'une quelconque des revendications précédentes 1 à 5 **caractérisé en ce que** l'élément de plateau (1) a une surface d'appui (2) latérale concave souple à faible module et une surface médiane opposée (3) qui est sensiblement plus dure et porte les deux chevilles de fixation (8) (9), et dans lequel la surface d'appui (2) souple à faible module s'étend autour de la périphérie de l'élément de plateau (1) et augmente son épaisseur pour fournir un bord déformable (5) pour simuler le bourrelet d'une cavité articulaire anatomique.

7. Composant glénoïde prosthétique selon l'une quelconque des revendications précédentes **caractérisé en ce que** la surface d'appui (2) est fabriquée à partir d'un polyuréthane élastomère souple.

8. Composant glénoïde prosthétique selon la revendication 7 **caractérisé en ce que** le polyuréthane élastomère souple a une valeur de dureté de 3,0 à 9,0 N/mm² d'après le procédé de test de dureté BS 2782 ; Pt 13 méthode 365D.

9. Composant glénoïde prosthétique selon la revendication 7 ou selon la revendication 8 **caractérisé en ce que** le polyuréthane élastomère souple est du Bionate 80A.

10. Composant glénoïde prosthétique selon l'une quelconque des revendications précédentes, **caractérisé en ce que** la surface médiane est fabriquée en un matériau polymère rigide.

11. Composant glénoïde prosthétique selon la revendication 10 **caractérisé en ce que** le matériau polymère rigide est du polyuréthane.

12. Composant glénoïde prosthétique selon la revendication 11 **caractérisé en ce que** le polyuréthane rigide a une valeur de dureté minimum de 65 N/mm² d'après la méthode de test de la dureté BS 2782 ; Pt 13 méthode 365D.

13. Composant glénoïde prosthétique selon la revendication 12 **caractérisé en ce que** ledit polyuréthane est du Bionate 75D.

14. Composant glénoïde prosthétique selon l'une quelconque des revendications précédentes **caractérisé en ce que** la surface médiane (3) est dotée d'une paire de rebords (12) (13) en projection situés au niveau du bord antérieur et postérieur ou à proximité.

15. Composant glénoïde prosthétique selon l'une quelconque des revendications précédentes **caractérisé en ce que** ladite surface médiane (3) est placée sur une partie de support (25) dont au moins une partie est fabriquée à partir d'une structure métallique trabéculaire poreuse.

16. Composant glénoïde prosthétique selon la revendication 15 **caractérisé en ce que** sensiblement la totalité de partie de support (25) est fabriquée comme une structure métallique trabéculaire poreuse.

17. Composant glénoïde prosthétique selon la revendication 15 **caractérisé en ce que** la partie de support (25) est fabriquée en PV avec des parties de structure métallique trabéculaire poreuse.

18. Composant glénoïde prosthétique selon l'une quelconque des revendications 15, 16 ou 17 **caractérisé en ce que** la structure métallique trabéculaire poreuse est fabriquée en déposant une première couche d'une poudre fabriquée à partir d'un métal choisi dans le groupe consistant en le titane, des alliages de titane, l'acier inoxydable, des alliages cobalt chrome, du tantale et du niobium, sur un substrat ; en faisant passer un faisceau laser au moins une fois sur ladite première couche de poudre, ledit faisceau laser ayant une puissance (P) en Joule par seconde avec une vitesse de balayage (v) en millimètres par seconde et un chevauchement de faisceau (b) en millimètres tels que le nombre calculé par la formule P/(b x v) se trouve dans la plage de 0,3-8 J/mm², ledit chevauchement de faisceau étant environ entre 0 % et -1 200 % pour donner la taille de pores requise ; en déposant au moins une couche de ladite poudre sur ladite première couche ; et en répétant lesdites étapes de balayage laser pour chaque couche successive jusqu'à ce qu'une hauteur de réseau désirée soit atteinte.

19. Composant glénoïde prosthétique selon l'une quelconque des revendications 15, 16 ou 17 **caractérisé en ce que** la structure métallique trabéculaire poreuse est fabriquée en déposant une première couche d'une poudre fabriquée à partir d'un métal choisi dans le groupe consistant en le titane, des alliages de titane, l'acier inoxydable, des alliages cobalt chrome, le tantale et le niobium, sur un substrat ; et en faisant passer un faisceau laser ayant une puissance (P) pendant une durée (µsec) avec une distance ponctuelle (µm), pour former une partie d'une pluralité de cellules unitaires prédéterminées dans ladite poudre métallique.
